(19) Europäisches Patentamt
European Patent Office
Office européen des brevets

(11) EP 2 415 795 B1

# (12) EUROPÄISCHE PATENTSCHRIFT

(45) Veröffentlichungstag und Bekanntmachung des Hinweises auf die Patenterteilung:
**13.11.2013 Patentblatt 2013/46**

(21) Anmeldenummer: **11176123.5**

(22) Anmeldetag: **01.08.2011**

(51) Int Cl.:
***C08G 18/02*** *(2006.01)* ***C08G 18/16*** *(2006.01)*
***C07C 263/16*** *(2006.01)* ***C07C 269/02*** *(2006.01)*
***C07C 271/02*** *(2006.01)* ***C07C 273/18*** *(2006.01)*
***C07C 275/60*** *(2006.01)* ***C07C 275/62*** *(2006.01)*
***C07F 9/54*** *(2006.01)*

## (54) Verfahren zur Herstellung von Polyisocyanaten und deren Verwendung

Method for manufacturing polyisocyanates and their application

Procédé de fabrication de polyisocyanates et leur utilisation

(84) Benannte Vertragsstaaten:
**AL AT BE BG CH CY CZ DE DK EE ES FI FR GB GR HR HU IE IS IT LI LT LU LV MC MK MT NL NO PL PT RO RS SE SI SK SM TR**

(30) Priorität: **03.08.2010 DE 102010038845**

(43) Veröffentlichungstag der Anmeldung:
**08.02.2012 Patentblatt 2012/06**

(73) Patentinhaber: **Bayer Intellectual Property GmbH**
**40789 Monheim (DE)**

(72) Erfinder:
- **Richter, Frank**
**51373 Leverkusen (DE)**
- **Brahm, Martin**
**51519 Odenthal (DE)**

(74) Vertreter: **BIP Patents**
**c/o Bayer Intellectual Property GmbH**
**Creative Campus Monheim**
**Alfred-Nobel-Straße 10**
**40789 Monheim (DE)**

(56) Entgegenhaltungen:
**EP-A1- 0 962 455 EP-A1- 1 645 577**
**WO-A1-02/48230**

Anmerkung: Innerhalb von neun Monaten nach Bekanntmachung des Hinweises auf die Erteilung des europäischen Patents im Europäischen Patentblatt kann jedermann nach Maßgabe der Ausführungsordnung beim Europäischen Patentamt gegen dieses Patent Einspruch einlegen. Der Einspruch gilt erst als eingelegt, wenn die Einspruchsgebühr entrichtet worden ist. (Art. 99(1) Europäisches Patentübereinkommen).

EP 2 415 795 B1

Printed by Jouve, 75001 PARIS (FR)

**Beschreibung**

Die Erfindung betrifft die Verwendung spezieller Phosphoniumsalze als Katalysatoren für die Isocyanatmodifizierung (Oligo- bzw. Polymerisierung) und ein Verfahren zur Herstellung entsprechend modifizierter Isocyanate.

Die Oligo- bzw. Polymerisierung von Isocyanaten, hier zusammenfassend Isocyanatmodifizierung genannt, ist seit langem bekannt. Enthalten die modifizierten Polyisocyanate freie NCO-Gruppen, die gegebenenfalls auch mit Blockierungsmitteln vorübergehend desaktiviert worden sein können, sind sie außerordentlich hochwertige Ausgangsstoffe für die Herstellung einer Vielzahl von Polyurethan-Kunststoffen und Beschichtungsmitteln.

Es haben sich eine Reihe technischer Verfahren zur Isocyanatmodifizierung etabliert, wobei man in der Regel das zu modifizierende Isocyanat, meist ein Diisocyanat, durch Zusatz von Katalysatoren umsetzt und diese anschließend, wenn der gewünschte Umsatzgrad des zu modifizierenden Isocyanates erreicht ist, durch geeignete Maßnahmen unwirksam macht (deaktiviert) und das erhaltene Polyisocyanat in der Regel vom nicht umgesetzten Monomer abtrennt. Eine Zusammenstellung dieser Verfahren des Standes der Technik findet sich in H. J. Laas et al., J. Prakt. Chem. 1994, 336, 185 ff.

Als Modifizierungskatalysatoren haben sich ionisch aufgebaute Verbindungen bewährt, da sie in sehr geringer Menge eingesetzt werden können und äußerst schnell zum gewünschten Ergebnis führen.

Die Möglichkeit, als Kation zum gegenüber Isocyanaten katalytisch aktiven Anion wie Hydroxid, Alkanoat, Alkoxylat etc. auch Tetraorganylphosphonium zu verwenden, ist allgemein bekannt, wenngleich in der Regel nicht explizit als besonders bevorzugt herausgehoben, vgl.: H. J. Laas et al., J. Prakt. Chem. 1994, 336, 185 ff.

Weiterhin ist die Verwendung von (Hydrogenpoly)fluoriden, optional auch in Form ihrer Phosphoniumsalze, sowie die Verwendung von Phosphoniumsalzen von Polyfluorsäuren für die Isocyanatmodifizierung u.a. bekannt aus den EP-A 962455, EP-A 962454, EP-A 896009, EP-A 798299, EP-A 447074, EP-A 379914, EP-A 339396, EP-A 315692, EP-A 295926, EP-A 235388, WO 02/48230 sowie EP-A 1645577.

Allerdings weisen die Tetraorganylphosphonium (Hydrogenpoly)fluoride des Standes der Technik bei der Durchführung der Modifizierungsreaktion den Nachteil auf, dass bei ihrer Verwendung die Reaktion mitunter nur durch kontinuierliche Katatalysatordosierung aufrecht zu erhalten ist, d.h. dass die Zersetzung des Katalysators im Isocyanatmilieu im Vergleich zur Modifizierungsreaktion technisch unakzeptabel schnell abläuft.

Der Erfindung lag die Aufgabe zugrunde, ein Modifizierverfahren unter Verwendung von Phosphoniumsalzen als Katalysatoren zu entwickeln, das nicht mit den vorgenannten Nachteilen behaftet sein sollte.

Dies ist durch die Bereitstellung des erfindungsgemäßen Verfahrens gelungen.

Gegenstand der Erfindung ist die Verwendung von Phosphoniumsalzen, die mindestens einen direkt an das P-Atom des Phosphoniumkations gebundenen Cycloalkylsubstituenten aufweisen, bei der Isocyanatmodifizierung.

Bevorzugte Phosphoniumsalze zur Isocyanatmodifizierung sind solche deren Kation der allgemeinen Formel I entsprechen:

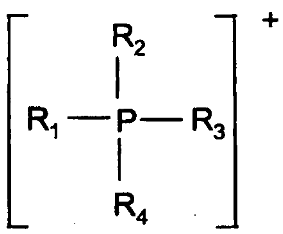

**Formel I**

wobei $R_1$ bis $R_4$ unabhängig voneinander für gleiche oder verschiedene, gegebenenfalls verzweigte und/oder substituierte organische Reste aus der Gruppe $C_1$- bis $C_{20}$-Alkyl, $C_3$- bis $C_{20}$-Cycloalkyl, $C_7$- bis $C_{20}$-Aralkyl und $C_6$- bis $C_{20}$-Aryl stehen, mit der Maßgabe, dass mindestens einer der Reste $R_1$ bis $R_4$ für einen gegebenenfalls verzweigten und/oder substituierten $C_3$- bis $C_{20}$-Cycloalkylrest steht und ein Kohlenstoffatom des Cycloalkylrings direkt mit dem P-Atom verbunden ist.

Bevorzugte Kationen der Formel I sind solche, bei denen $R_1$ bis $R_4$ unabhängig voneinander für gleiche oder verschiedene organische Reste aus der Gruppe $C_1$- bis $C_{20}$-Alkyl, Cyclopentyl und Cyclohexyl stehen, wobei die Alkylreste verzweigt und die Cycloalkylreste substituiert sein können,
mit der Maßgabe, dass mindestens zwei der Reste $R_1$ bis $R_4$ für einen gegebenenfalls substituierten Cyclopentyl- und/oder Cyclohexylrest stehen und diese jeweils über ein Ringkohlenstoffatom direkt mit dem P-Atom verbunden sind.

Besonders bevorzugte Phosphoniumsalze zur Isocyanatmodifizierung sind solche der oben genannten Art, wobei als Anionen X- zum Phosphonium-Kation der allg. Formel (I) folgende Spezies zum Einsatz kommen: Fluorid ($F^-$), Di- und/oder Poly(hydrogen)fluoride ($[F^- x (HF)m]$, wobei m für ganze oder gebrochene Zahlen von 0,001 bis 20, bevorzugt 0,1 bis 20, besonders bevorzugt 0,5 bis 20, ganz besonders bevorzugt für 0,5 bis 5 steht.

Die Katalysatoren können einzelnen oder in beliebigen Mischungen untereinander verwendet werden.

Ein weiterer Gegenstand der Erfindung ist ein Verfahren zur Isocyanatmodifizierung, bei dem

a) mindestens ein organisches Isocyanat mit einer NCO-Funktionalität ≥ 1,
b) ein Katalysator enthaltend mindestens ein erfindungsgemäß zu verwendendes Phosphoniumsalz,
c) optional Lösemittel und
d) optional Additive
zur Reaktion gebracht werden.

Mit dem erfindungsgemäßen Modifizierverfahren ist ganz allgemein eine breite Palette qualitativ hochwertiger und deshalb für den Polyurethansektor sehr wertvoller Polyisocyanate in einfacher Weise zugänglich. Abhängig vom verwendeten Ausgangs(di)isocyanat entstehen beim erfindungsgemäßen Verfahren Polyisocyanate vom sog. Isocyanat-Trimertyp (d.h. enthaltend Isocyanurat- und/oder Iminooxadiazindionstrukturen) mit geringem Anteil an Uretdiongruppen (Isocyanat-Dimere). Bei steigender Reaktionstemperatur steigt der Anteil letzterer in den Verfahrensprodukten an.

Zur Durchführung des erfindungsgemäßen Verfahrens können prinzipiell alle bekannten Mono-, Di- oder Polyisocyanate des Standes der Technik einzeln oder in beliebigen Mischungen untereinander eingesetzt werden.

Beispielhaft seien genannt: Hexamethylendiisocyanat (HDI), 2-Methylpentan-1,5-diisocyanat, 2,4,4-Trimethyl-1,6-hexandiisocyanat, 2,2,4-Trimethyl-1,6-hexandiisocyanat , 4-Isocyanatomethyl-1,8-octandiisocyanat, 3 (4)-Isocyanatomethyl-1 -methylcyclohexylisocyanat (IMCI), Isophorondiisocyanat (IPDI), 1,3- sowie 1,4-Bis(isocyanatomethyl) benzen (XDI), 1,3- sowie 1,4-Bis(isocyanatomethyl)cyclohexan (H6XDI), 2,4- sowie 2,6-Toluylendiisocyanat (TDI), Bis (4-isocyanatophenyl)methan (4,4'MDI), 4-Isocyanatophenyl-2-isocyanatophenylmethan (2,4'MDI) sowie mehrkernige Produkte, die durch Formaldehyd-Anilin-Polykondensation und anschließende Überführung der resultierenden (Poly) amine in die entsprechenden (Poly)isocyanate zugänglich sind (Polymer-MDI).

Bevorzugt ist die Verwendung von aliphatischen, cycloaliphatischen oder araliphatischen Di- oder Polyiisocyanaten einer Funktionalität ≥ 2.

Besonders bevorzugt ist die Verwendung von aliphatischen, cycloaliphatischen oder araliphatischen Diisocyanaten.

Ganz besonders bevorzugt sind Hexamethylendiisocyanat (HDI), 2-Methylpentan-1,5-diisocyanat, 2,4,4-Trimethyl-1,6-hexandiisocyanat, 2,2,4-Trimethyl-1,6-hexandiisocyanat, 4-Isocyanatomethyl-1,8-octandiisocyanat, 3 (4)-Isocyanatomethyl-1 -methylcyclohexylisocyanat (IMCI), Isophorondiisocyanat (IPDI), 1,3- sowie 1,4-Bis(isocyanatomethyl)benzen (XDI), 1,3- sowie 1,4-Bis(isocyanatomethyl)cyclohexan (H6XDI).

Es ist belanglos, nach welchen Verfahren die vorstehend genannten Isocyanate generiert werden, d.h. mit oder ohne Verwendung von Phosgen.

Die Menge des im erfindungsgemäßen Verfahren einzusetzenden Katalysators richtet sich in erster Linie nach dem verwendeten Isocyanat und der angestrebten Reaktionsgeschwindigkeit und liegt im Bereich 0,001 bis 5 mol-%, bezogen auf die Summe der Stoffmengen des eingesetzten Isocyanates und des Katalysators. Bevorzugt werden 0,002 bis 2 mol-% Katalysator eingesetzt.

Der Katalysator kann im erfindungsgemäßen Verfahren unverdünnt oder in Lösungsmitteln gelöst eingesetzt werden. Als Lösungsmittel kommen dabei alle Verbindungen in Frage, die nicht mit dem Katalysator reagieren und ihn in ausreichendem Maße zu lösen vermögen, z.B. aliphatische oder aromatische Kohlenwasserstoffe, Alkohole, Ketone, Ester sowie Ether. Bevorzugt werden Alkohole verwendet.

Das erfindungsgemäße Verfahren kann im Temperaturbereich von 0 °C bis + 250 °C, bevorzugt 20 bis 180 °C, besonders bevorzugt 40 bis 150 °C erfolgen und bei beliebigen Umsetzungsgraden, bevorzugt nachdem 5 bis 80 %, besonders bevorzugt 10 bis 60 %, des eingesetzten monomeren Diisocyanates umgesetzt wurden, abgebrochen werden.

Zur Katalysatordeaktivierung zwecks Abbruch der Reaktion bieten sich prinzipiell eine ganze Reihe vorbeschriebener Methoden des Standes der Technik an, wie z.B. die Zugabe (unter- oder über-) stöchiometrischer Mengen an Säuren oder Säurederivaten (z.B. Benzoylchlorid, saure Ester Phosphor oder Schwefel enthaltender Säuren, diese Säuren selbst etc., nicht jedoch HF), adsorptive Bindung des Katalysators und anschließende Abtrennung durch Filtration, usw.

Im Anschluss an die Katalysatordesaktivierung kann das nicht umgesetzte Monomer sowie gegebenenfalls mitverwendetes Lösemittel mit Hilfe aller bekannten Separationstechniken wie z.B. Destillation, gegebenenfalls in der speziellen Ausführungsform der Dünnschichtdestillation Extraktion oder Kristallisation/Filtration abgetrennt werden. Selbstverständlich können auch Kombinationen zweier oder mehrerer dieser Techniken angewendet werden.

Soll das erfindungsgemäß hergestellte Polyisocyanat noch freies, nicht umgesetztes Monomer enthalten, wie es z.B. für die Weiterverarbeitung zu NCO-blockierten Produkten von Interesse ist, so kann nach Katalysatordesaktivierung auf die Monomerenabtrennung verzichtet werden.

Bevorzugt wird das nicht umgesetzte Monomer abgetrennt. Bevorzugt weisen die erfindungsgemäßen Produkte

nach der Abtrennung einen Restmonomergehalt < 0, 5 % bevorzugt < 0,1 Gew.- % auf.

Bevorzugt wird das nicht umgesetzte Monomer destillativ abgetrennt.

Verglichen mit der Katalyse durch quaternäre Phosphoniumsalze, die Aryl- und/oder geradkettige Alkylgruppen am P-Atom aufweisen, z.B. Triphenyl-butyl-, Tetra-n-butyl- oder Tetra-n-octylphosphoniumsalze (s. Vergleichsbeispiele), beobachtet man im erfindungsgemäßen Verfahren bei sonst gleichen Reaktionsbedingungen eine deutlich verbesserte Katalysatorstandzeit - die mit Temperatursteigerung nicht so stark abfällt, wie bei den o.g. Verbindungen des Standes der Technik - in den Beispielen und Vergleichsbeispielen durch die Turnover Frequenz (TOF) belegt. Letztere ist bezogen auf die Stoffmenge der in der Oligomerisierung umgesetzten NCO-Gruppen A, die Stoffmenge an dafür nötigem Katalysator B und die Reaktionszeit t gemäß folgender Gleichung definiert:

$$TOF = A*(B*t)^{-1}\ [mol*(mol*sec)^{-1}]\ .$$

Nach einer besonderen, kontinuierlich arbeitenden Ausführungsform des erfindungsgemäßen Verfahrens kann die Oligomerisierung in einem Rohrreaktor bzw. einer Mehrkesselkaskade vorgenommen werden. Hierbei profitiert man besonders von der signifikant geringeren Neigung der erfindungsgemäßen Katalysatoren im Vergleich zu den bekannten Katalysatoren des Standes der Technik, selbst bei Applikation in hochkonzentrierter Lösung bzw. als reiner Wirkstoff, spontan Gelteilchen im Produkt zu bilden.

Die nach dem erfindungsgemäßen Verfahren erhältlichen Produkte bzw. Produktgemische stellen mithin vielseitig verwendbare Ausgangsmaterialien zur Herstellung von, gegebenenfalls geschäumte(n), Kunststoffe(n) sowie Lacken, Beschichtungsmitteln, Klebstoffen und Zuschlagstoffen dar.

Die erfindungsgemäßen Verfahrensprodukte können rein oder in Verbindung mit anderen Isocyanatderivaten des Standes der Technik, wie z.B. Uretdion-, Biuret-, Allophanat-, Isocyanurat- und/oder Urethan-Gruppen enthaltenden Polyisocyanaten, deren freie NCO-Gruppen gegebenenfalls mit Blockierungsmitteln desaktiviert wurden, eingesetzt werden.

Die nachfolgenden Vergleichsbeispiele und Beispiele sollen die Erfindung näher erläutern, ohne sie jedoch einzuschränken.

**Beispiele**

Alle Prozentangaben sind, soweit nicht anders vermerkt, als Gewichtsprozent zu verstehen.

Mol-% Angaben wurden NMR-spektroskopisch ermittelt und beziehen sich immer, so nicht anders ausgewiesen, auf die Summe der NCO-Folgeprodukte. Die Messungen erfolgten auf den Geräten DPX 400 bzw. DRX 700 der Fa. Brucker an ca. 5 %igen ($^1$H-NMR) bzw. ca. 50 %igen ($^{13}$C-NMR) Proben in trockenem $C_6D_6$ bei einer Frequenz von 400 bzw. 700 MHz ($^1$H-NMR) oder 100 bzw. 176 MHz ($^{13}$C-NMR). Als Referenz für die ppm-Skale wurden geringe Mengen von Tetramethylsilan im Lösungsmittel mit 0 ppm $^1$H-NMR-chem. Verschiebung heran gezogen. Alternativ wurde auf das Signal des im Lösungsmittel enthaltenen $C_6D_5H$ referenziert: 7,15 ppm $^1$H-NMR-chem. Verschiebung, 128,02 ppm $^{13}$C-NMR-chem. Verschiebung.. Daten für die chemische Verschiebung der in Frage kommenden Verbindungen wurden der Literatur entnommen (vgl. D. Wendisch, H. Reiff und D. Dieterich, Die Angewandte Makromolekulare Chemie 141, 1986, 173-183 und darin zit. Lit sowie EP-A 96 009.

Die dynamischen Viskositäten wurden bei 23 °C mit dem Viskosimeter VT 550 der Fa. Haake bestimmt. Durch Messungen bei unterschiedlichen Schergeschwindigkeiten wurde sichergestellt, daß das Fließverhalten der beschriebenen erfindungsgemäßen Polyisocyanatmischungen wie auch das der Vergleichsprodukte dem idealer Newtonscher Flüssigkeiten entspricht. Die Angabe der Schergeschwindigkeit kann deshalb entfallen.

Die Bestimmung der Restmonomergehalte erfolgte gaschromatographisch.

Alle Reaktionen wurden, wenn nicht anders angegeben, unter einer Stickstoffatmosphäre durchgeführt.

Die verwendeten Diisocyanate sind Produkte der Bayer MaterialScience AG, D-51368 Leverkusen, alle anderen kommerziell zugänglichen Chemikalien wurden von der Fa. Aldrich, D-82018 Taufkirchen, bezogen.

Die Katalysatoren wurden nach literaturbekannten Methoden gemäß Schema 1 gewonnen.

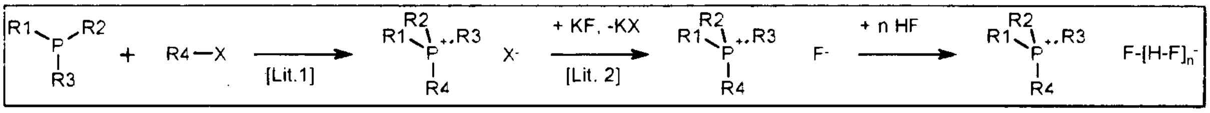

Schema 1

Lit. 1: K. Jödden in Methoden der Organischen Chemie (Houben Weyl) Band E1, S. 495 ff und darin zit. Lit.;

Lit. 2: S. Dermeik and Y. Sasson, *J. Org. Chem.* 54 **1989**, 4827-4829 und darin zit. Lit.

**Beispiele 1a bis 1d - Vergleichsbeispiele**

1a) Katalysator: Triphenyl-n-butylphosphonium(hydrogen)difluorid (R1-R3 = Ph, R4 =n-Bu; n =1)

1b) Katalysator: Tetra-n-butylphosphonium(hydrogen)difluorid (R1-R4 = n-Bu; n =1)

1c) Katalysator: Tetra-n-butylphosphonium(dihydrogen)trifluorid (R1-R4 = n-Bu; n =2)

1d) Katalysator: Tetra-n-octylphosphonium(hydrogen)difluorid (R1-R4 = n-Oct; n =1)

In einem doppelwandigen, durch einen externen Kreislauf auf die jeweils gewünschte Starttemperatur temperierten Planschliffgefäß mit Rührer, an eine Inertgasanlage (Stickstoff/Vakuum) angeschlossenem Rückflusskühler und Thermometer wurden 1000 g HDI vorgelegt und durch einstündiges Rühren im Vakuum (0,1 mbar) von gelösten Gasen befreit. Nach Belüften mit Stickstoff wurde die in Tabelle 1 angegebene Katalysatormenge (als 70%ige Lösung in Isopropanol) so dosiert, dass die in Tabelle 1 angegebene Maximaltemperatur nicht überschritten wurde. Nachdem 1 mol NCO Gruppen umgesetzt waren, wurde der Katalysator durch Zugabe einer zum Katalysator äquivalenten Menge an p-Toluolsulfonsäure (als 40%ige Lösung in Isopropanol) deaktiviert, weitere 30 min bei Reaktionstemperatur nachgerührt und anschließend aufgearbeitet. Die Zeit zwischen erster Katalysatorzugabe und Zugabe der Deaktivatorlösung wurde zur Berechnung der in Tabelle 1 angegebenen TOF (turnover frequency, [mol umgesetzte NCO-Gruppen / (mol Katalysator * Reaktionszeit in Sekunden)]) herangezogen.

Die Aufarbeitung erfolgte durch Vakuumdestillation in einem Dünnschichtverdampfer, Typ Kurzwegverdampfer (KWV), mit vorgeschaltetem Vorverdampfer (VV) (Destillationsdaten: Druck: 0,08 +/- 0,04 mbar, VV-Temperatur: 120°C, HV-Temp.: 140°C), wobei nicht umgesetztes Monomer als Destillat und das monomerenarme Polyisocyanatharz als Sumpfprodukt separiert wurden (Startdurchlauf: Beispiel 1-0). Das Polyisocyanatharz wurde analysiert, Ergebnisse s. Tabelle 2, das Destillat wurde in einer zweiten Planschliff-Rührapparatur, die identisch zur ersten aufgebaut ist, gesammelt und mit frisch entgastem HDI auf die Ausgangsmenge (1000 g) aufgefüllt. Anschließend wurde erneut katalysiert und verfahren wie eingangs beschrieben. Diese Verfahrensweise wurde unter Variation der Reaktionstemperatur mehrmals wiederholt. Die Ergebnisse sind Tabelle 1 zu entnehmen.

In Falle des anteilig mit Phenylgruppen substituierten Katalysators wurde auf eine Durchführung der Reaktion bei höherer Reaktionstemperatur als 60°C verzichtet, da die Katalysatorzersetzung bereits bei 60°C derartig schnell vonstatten ging, dass unakzeptabel hohe Katalysatormengen zur Aufrechterhaltung der Reaktion bis zum gewünschten Umsatz (hier: 1 mol NCO-Gruppen) benötigt wurden. Aber auch bei den ausschließlich mit Alkylgruppen substituierten Phosphoniumsalzen ist bei höheren Reaktionstemperaturen eine signifikante Verringerung der Katalysatoraktivität, erkennbar an den geringeren TOF-Werten, zu verzeichnen.

Tabelle 1

| Beispiel-Nr. | | Katalysator- | | Reaktionstemperatur [°C] | | TOF |
|---|---|---|---|---|---|---|
| | | kation | anion | Start | Max. | |
| | | | | | | |
| 1a- | A | $n-Bu(Ph)_3P^+$ | $[HF_2]^-$ | 60 | 64 | 0,007 |
| 1a- | B | $n-Bu(Ph)_3P^+$ | $[HF_2]^-$ | 60 | 65 | 0,005 |
| | | | | | | |
| 1b- | A | $n-Bu_4P^+$ | $[HF_2]^-$ | 60 | 62 | 0,61 |
| 1b- | B | $n-Bu_4P^+$ | $[HF_2]^-$ | 60 | 63 | 0,96 |
| 1b- | C | $n-Bu_4P^+$ | $[HF_2]^-$ | 80 | 84 | 0,42 |
| 1b- | D | $n-Bu_4P^+$ | $[HF_2]^-$ | 100 | 105 | 0,11 |

(fortgesetzt)

| Beispiel-Nr. | | Katalysator- | | Reaktionstemperatur [°C] | | TOF |
|---|---|---|---|---|---|---|
| | | kation | anion | Start | Max. | |
| 1b- | E | n-$Bu_4P^+$ | $[HF_2]^-$ | 120 | 127 | 0,05 |
| 1b- | F | n-$Bu_4P^+$ | $[HF_2]^-$ | 140 | 152 | 0,04 |
| | | | | | | |
| 1c- | A | n-$Bu_4P^+$ | $[H_2F_3]^-$ | 60 | 62 | 0,74 |
| 1c- | B | n-$Bu_4P^+$ | $[H_2F_3]^-$ | 60 | 65 | 1,04 |
| 1c- | C | n-$Bu_4P^+$ | $[H_2F_3]^-$ | 80 | 81 | 0,43 |
| 1c- | D | n-$Bu_4P^+$ | $[H_2F_3]^-$ | 100 | 107 | 0,09 |
| 1c- | E | n-$Bu_4P^+$ | $[H_2F_3]^-$ | 120 | 121 | 0,06 |
| 1c- | F | n-$Bu_4P^+$ | $[H_2F_3]^-$ | 140 | 140 | 0,05 |
| | | | | | | |
| 1d- | A | n-$Oct_4P^+$ | $[HF_2]^-$ | 60 | 62 | 0,48 |
| 1d- | B | n-$Oct_4P^+$ | $[HF_2]^-$ | 60 | 36 | 0,73 |
| 1d- | C | n-$Oct_4P^+$ | $[HF_2]^-$ | 80 | 34 | 0,32 |
| 1d- | D | n-$Oct_4P^+$ | $[HF_2]^-$ | 100 | 34 | 0,27 |
| 1d- | E | n-$Oct_4P^+$ | $[HF_2]^-$ | 120 | 34 | 0,08 |
| 1d- | F | n-$Oct_4P^+$ | $[HF_2]^-$ | 140 | 34 | 0,03 |

**Beispiel 2 erfindungsgemäß**

Katalysator: n-Butyl-tricyclopentylphosphonium(hydrogen)difluorid

Es wurde Verfahren wie eingangs beschrieben, mit dem Unterschied, dass eine 40%ige n-Butyl-tricyclopentylphosphonium(hydrogen)difluoridlösung in Isopropanol als Katalysator verwendet wurde. Die Ergebnisse sind Tabelle 2 zu entnehmen.

Tabelle 2

| Beispiel-Nr. | | Reaktionstemperatur [°C] | | TOF |
|---|---|---|---|---|
| | | Start | Max. | |
| 2- | A | 60 | 64 | 2,14 |
| 2- | B | 60 | 68 | 3,12 |
| 2- | C | 80 | 99 | 6,45 |
| 2- | D | 100 | 109 | 3,67 |
| 2- | E | 120 | 134 | 1,47 |
| 2- | F | 140 | 155 | 1,09 |
| 2- | G | 30 | 35 | 1,78 |

**Beispiel 3 erfindungsgemäß**

Katalysator: n-Butyl-tricyclopentylphosphonium(dihydrogen)trifluorid

Es wurde Verfahren wie im Vergleichsbeispiel 1 beschrieben, mit dem Unterschied, dass eine 45%ige n-Butyl-tricyclopentylphosphonium(dihydrogen)trifluoridlösung in Isopropanol als Katalysator verwendet wurde. Die Ergebnisse sind den Tabelle 3 zu entnehmen.

Tabelle 3

| Beispiel-Nr. | | Reaktionstemperatur [°C] | | TOF |
|---|---|---|---|---|
| | | Start | Max. | |
| 3- | 0 | 60 | 64 | 2,41 |
| 3- | A | 60 | 68 | 3,22 |
| 3- | B | 80 | 84 | 7,36 |
| 3- | C | 100 | 105 | 4,22 |

**Beispiel 4 erfindungsgemäß**

Katalysator: n-Butyl-tricyclohexylphosphonium(hydrogen)difluorid

Es wurde Verfahren wie im Vergleichsbeispiel 1 beschrieben, mit dem Unterschied, dass eine 20 % ige n-Butyl-tricyclohexylphosphonium(hydrogen)difluoridlösung in Isopropanol/Diethylenglykoldimethylether (1:3,3) als Katalysator verwendet wurde. Die Ergebnisse sind Tabelle 4 zu entnehmen.

Tabelle 4

| Beispiel-Nr. | | Reaktionstemperatur [°C] | | TOF |
|---|---|---|---|---|
| | | Start | Max. | |
| 4- | A | 60 | 65 | 1,98 |
| 4- | B | 60 | 63 | 2,25 |
| 4- | C | 80 | 90 | 4,42 |
| 4- | D | 100 | 105 | 4,56 |
| 4- | E | 120 | 139 | 4,89 |
| 4- | F | 140 | 149 | 1,42 |

**Beispiel 5 erfindungsgemäß**

Katalysator: Di-n-butyl-dicyclopentylphosphonium(hydrogen)difluorid

Es wurde Verfahren wie im Vergleichsbeispiel 1 beschrieben, mit dem Unterschied, dass eine 50%ige Di-n-butyl-dicyclopentylphosphonium(hydrogen)difluoridlösung in Isopropanol als Katalysator verwendet wurde. Die Ergebnisse sind Tabelle 5 zu entnehmen.

Tabelle 5

| Beispiel-Nr. | | Reaktionstemperatur [°C] | | TOF |
|---|---|---|---|---|
| | | Start | Max. | |
| 5- | A | 60 | 61 | 1,15 |
| 5- | B | 60 | 61 | 1,96 |
| 5- | C | 80 | 83 | 1,08 |
| 5- | D | 100 | 103 | 0,52 |
| 5- | E | 120 | 128 | 0,35 |
| 5- | F | 140 | 146 | 0,11 |

**Beispiel 6 erfindungsgemäß**

Katalysator: Tri-n-hexyl-cyclohexylphosphonium(hydrogen)difluorid

Es wurde Verfahren wie im Vergleichsbeispiel 1 beschrieben, mit dem Unterschied, dass eine 90%ige Tri-n-hexyl-cyclohexylphosphonium(hydrogen)difluoridlösung in Isopropanol als Katalysator verwendet wurde. Die Ergebnisse sind Tabelle 6 zu entnehmen.

Tabelle 6

| Beispiel-Nr. | | Reaktionstemperatur [°C] | | TOF |
|---|---|---|---|---|
| | | Start | Max. | |
| 6- | 0 | 60 | 62 | 0,77 |
| 6- | A | 60 | 62 | 1,11 |
| 6- | B | 100 | 107 | 0,83 |
| 6- | C | 140 | 145 | 0,18 |

**Beispiele 7 und 8 (Vergleichsbeispiele):**

Katalysator: Tetra-n-butylphosphonium(hydrogen)difluorid

**sowie 9 und 10 (erfindungsgemäß)**

Katalysator: n-Butyl-tricyclopentylphosphonium(hydrogen)difluorid

Es wurde Verfahren wie im Vergleichsbeispiel 1 beschrieben, mit dem Unterschied, dass an Stelle HDI IPDI eingesetzt wurde und der Umsatz an NCO Gruppen auf ca. 2 mol eingestellt wurde. Bei 60°C Reaktionstemperatur (Vergleichsbeispiel 7) resultiert eine TOF zwischen 0,002 und 0,005. Wird die Reaktionstemperatur auf 100°C angehoben (Vergleichsbeispiel 8), lässt sich auch trotz kontinuierlicher Katalysatorzugabe keine gleichmäßige Reaktionsführung erreichen. Nachdem 1,26 mol an NCO-Gruppen umgesetzt worden waren betrug die TOF 0,0005.

Bei analog Vergleichsbeispiel 7 bzw. 8 geführten Reaktionen mit dem Katalysator aus Beispiel 2 (einmalige Zugabe) wurden erhalten:

60°C (Beispiel 9): TOF im Bereich 0,02 bis 0,06
100°C (Beispiel 10): TOF ca. 0,01

## Patentansprüche

1. Verwendung von Phosphoniumsalzen, die mindestens einen direkt an das P-Atom des Phosphoniumkations gebundenen Cycloalkylsubstituenten aufweisen, bei der Isocyanatmodifizierung.

2. Verwendung gemäß Anspruch 1, **dadurch gekennzeichnet, dass** Phosphoniumsalze eingesetzt werden, deren Kation der allgemeinen Formel I entspricht:

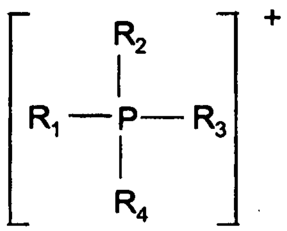

Formel I

wobei $R_1$ bis $R_4$ unabhängig voneinander für gleiche oder verschiedene, gegebenenfalls verzweigte und/oder substituierte organische Reste aus der Gruppe $C_1$- bis $C_{20}$-Alkyl, $C_3$-bis $C_{20}$-Cycloalkyl, $C_7$- bis $C_{20}$-Aralkyl und $C_6$- bis

$C_{20}$-Aryl stehen,
mit der Maßgabe, dass mindestens einer der Reste $R_1$ bis $R_4$ für einen gegebenenfalls verzweigten und/oder substituierten $C_3$- bis $C_{20}$-Cycloalkylrest steht und ein Kohlenstoffatom des Cycloalkylrings direkt mit dem P-Atom verbunden ist.

**3.** Verwendung gemäß Anspruch 2, **dadurch gekennzeichnet, dass** die Kationen der Formel I solche sind, bei denen $R_1$ bis $R_4$ unabhängig voneinander für gleiche oder verschiedene organische Reste aus der Gruppe $C_1$- bis $C_{20}$-Alkyl, Cyclopentyl und Cyclohexyl stehen, wobei die Alkylreste verzweigt und die Cycloalkylreste substituiert sein können, mit der Maßgabe, dass mindestens zwei der Reste $R_1$ bis $R_4$ für einen gegebenenfalls substituierten Cyclopentyl- und/oder Cyclohexylrest stehen und diese jeweils über ein Ringkohlenstoffatom direkt mit dem P-Atom verbunden sind.

**4.** Verwendung gemäß Anspruch 2 oder 3, **dadurch gekennzeichnet, dass** das Gegenion $X^-$ zum Phosphonium-Kation der allg. Formel (I) Fluorid ($F^-$) oder ein Di- oder Poly(hydrogen)fluorid ([F- x $(HF)_m$] ist, wobei m für ganze oder gebrochene Zahlen von 0,001 bis 20 steht.

**5.** Verwendung gemäß einem der Ansprüche 1 bis 4, **dadurch gekennzeichnet, dass** aliphatische, cycloaliphatische oder araliphatische Di- oder Polyiisocyanate einer Funktionalität ≥ 2 eingesetzt werden.

**6.** Verfahren zur Isocyanatmodifizierung, bei dem

a) mindestens ein organisches Isocyanat mit einer NCO-Funktionalität ≥ 1,
b) ein Katalysator enthaltend mindestens ein Phosphoniumsalz, welches dem jenigen entspricht, das im einem der Ansprüche 1-5 verwendet wird,
c) optional Lösemittel und
d) optional Additive

zur Reaktion gebracht werden.

**7.** Verfahren gemäß Anspruch 6, **dadurch gekennzeichnet, dass** aliphatische, cycloaliphatische oder araliphatische Di- oder Polyisocyanate einer Funktionalität ≥ 2 eingesetzt werden.

**8.** Verfahren gemäß Anspruch 6 oder 7, **dadurch gekennzeichnet, dass** das Verfahren im Temperaturbereich von 0 °C bis + 250°C durchgeführt wird.

**9.** Verfahren gemäß einem der Ansprüche 6 bis 8, **dadurch gekennzeichnet, dass** das Verfahren nachdem 5 bis 80 % des eingesetzten monomeren Diisocyanates umgesetzt wurden, abgebrochen wird.

**10.** Verfahren gemäß einem der Ansprüche 6 bis 9, **dadurch gekennzeichnet, dass** das nicht umgesetzte Monomer aus der Reaktionsmischung abgetrennt wird.

## Claims

**1.** Use of phosphonium salts which have at least one cycloalkyl substituent bonded directly to the P atom of the phosphonium cation, in isocyanate modification.

**2.** Use according to Claim 1, **characterized in that** phosphonium salts are used, the cation of which corresponds to the general formula I:

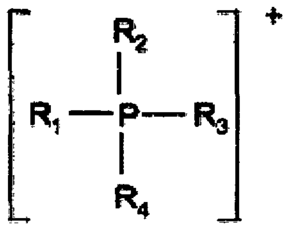

formula I

where $R_1$ to $R_4$, independently of one another, are identical or different, optionally branched and/or substituted organic radicals from the group $C_1$-to $C_{20}$-alkyl, $C_3$- to $C_{20}$-cycloalkyl, $C_7$- to $C_{20}$-aralkyl and $C_6$- to $C_{20}$-aryl, with the proviso that at least one of the radicals $R_1$ to $R_4$ is an optionally branched and/or substituted $C_3$- to $C_{20}$-cycloalkyl radical and one carbon atom of the cycloalkyl ring is bonded directly to the P atom.

3. Use according to Claim 2, **characterized in that** the cations of the formula I are those in which $R_1$ to $R_4$, independently of one another, are identical or different organic radicals from the group $C_1$- to $C_{20}$-alkyl, cyclopentyl and cyclohexyl, where the alkyl radicals may be branched and the cycloalkyl radicals may be substituted, with the proviso that at least two of the radicals $R_1$ to $R_4$ are an optionally substituted cyclopentyl and/or cyclohexyl radical and these are in each case bonded directly to the P atom via a ring carbon atom.

4. Use according to Claim 2 or 3, **characterized in that** the counterion X- to the phosphonium cation of the general formula (I) is fluoride ($F^-$) or a di- or poly(hydrogen)fluoride ([F- x $(HF)_m$], where m is an integer or fraction from 0.001 to 20.

5. Use according to one of Claims 1 to 4, **characterized in that** aliphatic, cycloaliphatic or araliphatic di- or polyisocyanates with a functionality ≥ 2 are used.

6. Method for isocyanate modification, in which

   a) at least one organic isocyanate with an NCO functionality ≥ 1,
   b) a catalyst containing at least one phosphonium salt which corresponds to that which is used in one of Claims 1-5,
   c) optionally solvent and
   d) optionally additives

   are reacted.

7. Method according to Claim 6, **characterized in that** aliphatic, cycloaliphatic or araliphatic di- or polyisocyanates with a functionality ≥ 2 are used.

8. Method according to Claim 6 or 7, **characterized in that** the method is carried out in the temperature range from 0°C to +250°C.

9. Method according to one of Claims 6 to 8, **characterized in that** the method is terminated after 5 to 80% of the monomeric diisocyanate used have been reacted.

10. Method according to one of Claims 6 to 9, **characterized in that** the unreacted monomer is separated off from the reaction mixture.

**Revendications**

1. Utilisation de sels de phosphonium, qui comportent au moins un substituant cycloalkyle lié directement à l’atome de phosphore du cation phosphonium, dans la modification d’isocyanates.

2. Utilisation selon la revendication 1, **caractérisée en ce qu'**on utilise des sels de phosphonium dont le cation correspond à la formule générale I :

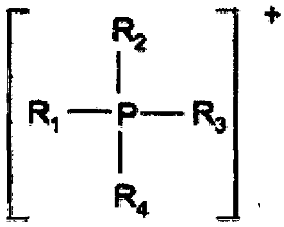

formule I

dans laquelle $R_1$ à $R_4$ représentent indépendamment les uns des autres des radicaux organiques identiques ou différents, éventuellement ramifiés et/ou substitués, choisis dans le groupe constitué par des radicaux alkyle en $C_1$-$C_{20}$, cycloalkyle en $C_3$-$C_{20}$, aralkyle en $C_7$-$C_{20}$ et aryle en $C_6$-$C_{20}$,
étant entendu qu'au moins l'un des radicaux $R_1$ à $R_4$ représente un radical cycloalkyle en $C_3$-$C_{20}$ éventuellement ramifié et/ou substitué et un atome de carbone du cycle cycloalkyle est lié directement à l'atome de phosphore.

3. Utilisation selon la revendication 2, **caractérisée en ce que** les cations de formule I sont ceux dans lesquels $R_1$ à $R_4$ représentent, indépendamment les uns des autres, des radicaux organiques identiques ou différents choisis dans le groupe constitué par les radicaux alkyle en $C_1$-$C_{20}$, cyclopentyle et cyclohexyle, les radicaux alkyle pouvant être ramifiés et les radicaux cycloalkyle pouvant être substitués, étant entendu qu'au moins deux des radicaux $R_1$ à $R_4$ représentent un radical cyclopentyle et/ou un radical cyclohexyle éventuellement substitués et ceux-ci sont liés directement par un atome de carbone formant le cycle à l'atome de phosphore.

4. Utilisation selon la revendication 2 ou 3, **caractérisée en ce que** l'ion $X^-$ opposé au cation phosphonium de formule générale (I) est un fluorure ($F^-$) ou un di- ou poly(hydrogéno) fluorure ($[F^- \times (HF)_m]$ , m représentant des nombres entiers ou fractionnaires valant de 0,001 à 20.

5. Utilisation selon l'une quelconque des revendications 1 à 4, **caractérisée en ce qu'**on utilise des di- ou polyisocyanates aliphatiques, cycloaliphatiques ou araliphatiques ayant une fonctionnalité ≥ 2.

6. Procédé pour la modification d'isocyanates, dans lequel on met en réaction

   a) au moins un isocyanate organique ayant une fonctionnalité NCO ≥ 1,
   b) un catalyseur contenant au moins un sel de phosphonium, qui correspond à celui qui est utilisé dans l'une quelconque des revendications 1 à 5,
   c) en option un solvant et
   d) en option des additifs.

7. Procédé selon la revendication 6, **caractérisé en ce qu'**on utilise des di- ou polyisocyanates aliphatiques, cycloaliphatiques ou araliphatiques ayant une fonctionnalité ≥ 2.

8. Procédé selon la revendication 6 ou 7, **caractérisé en ce que** le procédé est effectué dans la plage de température de 0 °C à +250 °C.

9. Procédé selon l'une quelconque des revendications 6 à 8, **caractérisé en ce qu'**on interrompt le processus après que 5 à 80 % du diisocyanate monomère utilisé ont réagi.

10. Procédé selon l'une quelconque des revendications 6 à 9, **caractérisé en ce qu'**on sépare du mélange réactionnel le monomère n'ayant pas réagi.

## IN DER BESCHREIBUNG AUFGEFÜHRTE DOKUMENTE

*Diese Liste der vom Anmelder aufgeführten Dokumente wurde ausschließlich zur Information des Lesers aufgenommen und ist nicht Bestandteil des europäischen Patentdokumentes. Sie wurde mit größter Sorgfalt zusammengestellt; das EPA übernimmt jedoch keinerlei Haftung für etwaige Fehler oder Auslassungen.*

### In der Beschreibung aufgeführte Patentdokumente


- EP 962455 A
- EP 962454 A
- EP 896009 A
- EP 798299 A
- EP 447074 A
- EP 379914 A
- EP 339396 A
- EP 315692 A
- EP 295926 A
- EP 235388 A
- WO 0248230 A
- EP 1645577 A
- EP 96009 A


### In der Beschreibung aufgeführte Nicht-Patentliteratur


- **H. J. LAAS et al.** *J. Prakt. Chem.,* 1994, vol. 336, 185 ff
- **H. J. LAAS et al.** *J. Prakt. Chem.,* 1994, vol. 336, 185 ff
- **D. WENDISCH ; H. REIFF ; D. DIETERICH.** *Die Angewandte Makromolekulare Chemie,* 1986, vol. 141, 173-183